# EUROPEAN PATENT APPLICATION

(11) **EP 3 078 397 A1**
(43) Date of publication of application: **12.10.2016**
(21) Application number: 15163100.9
(22) Date of filing: 10.04.2015
(51) Int. Cl.: A61Q 17/04, A61K 8/81

(54) **TOPICAL SUNSCREEN COMPOSITIONS**

(71) Applicant: DSM IP Assets B.V., 6411 TE Heerlen (NL)
(72) Inventor: DESHAYES, Cyrille, 4303 Kaiseraugst (CH); HECKER, Karina, 4303 Kaiseraugst (CH); KLOCK, Jochen, 4303 Kaiseraugst (CH); MENDROK-EDINGER, Christine, 4303 Kaiseraugst (CH)
(74) Representative: Berg, Katja

(57) **Abstract**

The invention relates to topical sunscreen compositions comprising an acrylic emulsion polymer and at least one UV-filter substance. The compositions provide increased SPF performance, improved water resistance without being unpleasantly sticky.

## Description

The invention relates to topical sunscreen compositions comprising an acrylic emulsion polymer and at least one UV-filter substance. The compositions provide increased SPF performance, improved water resistance without being unpleasantly sticky.

Sun care products have evolved considerably over the years. Earlier formulations were intended to protect the user from UV-B radiation as was once thought that UV-B rays were the most important contributors to wrinkling, skin disease, and skin cancer. However, more recent studies have shown that UV-A radiation is equally or even more important in the development of solar damage and skin diseases, such as lupus erythematosus and melanoma and non-melanoma skin cancer. Thus, today's focus is towards eliminating as much of UVA (320-400 nm) and / or UVB (280-320 nm) light as possible. Consequently, there's a constantly increasing need for sun care products exhibiting high SPF's (Sun Protection Factor) and high UVA protection.

Water resistance of sun care products is a key parameter for today's sunscreens and can for example be achieved by the addition of film forming polymers. These film forming polymers, however, are either not sufficiently effective and/ or render the product unattractive for the end consumer due to the resulting sensory properties as such products often exhibit an oily, dull, and sticky feeling on the skin as e.g. outlined in DE 102010063825.

It was therefore the object of the present invention to remedy the disadvantages of the prior art and to develop sunscreen compositions which exhibit high SPF's as well as an improved water resistance and thus provide excellent UV-protection in the UVA and UVB range even after bathing. Furthermore, such compositions should exhibit excellent sensory properties, in particular without being unpleasantly sticky.

Surprisingly it has been found, that the SPF as well as the water resistance of UV-filter substances in topical sunscreen compositions can be significantly improved by the addition of a specific acrylic emulsion polymer without negatively affecting the sensory properties.

Thus, in one embodiment the invention relates to a topical sunscreen composition comprising at least 1 wt.-% of at least one UV-filter substance and an effective amount of an acrylic emulsion polymer obtained by emulsion polymerization of a monomer composition consisting of a mixture of methacrylic acid, ethyl acrylate and n-butyl methacrylate.

Furthermore, the invention relates to the use of an acrylic emulsion polymer obtained by emulsion polymerization of a monomer composition consisting of a mixture of methacrylic acid, ethyl acrylate and n-butyl methacrylate for increasing the water resistance and/ or SPF of a topical sunscreen composition comprising at least one UV-filter substance.

In a further aspect the invention relates to a method for increasing the water resistance and/ or the SPF of at least one UV-filter substance in a topical sunscreen composition, said method comprising the addition of an acrylic emulsion polymer obtained by emulsion polymerization of a monomer composition consisting of a mixture of methacrylic acid, ethyl acrylate and n-butyl methacrylate into said topical sunscreen composition and, preferably, observing or appreciating the result.

The term 'consisting of' as used according to the present invention means that the total amount of monomer ideally sum up to 100 wt.-%. It is however not excluded that small amount of impurities or additives may be present such as e.g. in amounts of less than 5 wt.-%, preferably less than 3 wt.-% which are e.g. introduced via the respective raw materials.

The term "effective amount" means an amount that can achieve the stated results. In particular, the term "effective amount" refers to a concentration (based on solids) of at least 0.05 wt.-% based on the total weight of the topical sunscreen composition. Preferably, a concentration of 0.1-3 wt.-%, more preferably of 0.5-2 wt.-%, most preferably of 0.5-1.5 wt.-% is used.

In all embodiments of the invention the monomer composition preferably consists of a mixture of 10-30 wt.-% of methacrylic acid, 5-15 wt.-% of ethyl acrylate and 60-80 wt.-% of n-butyl methacrylate, more preferably of a mixture of 10-30 wt.-% of methacrylic acid, 5-15 wt.-% of ethyl acrylate and 60-80 wt.-% of n-butyl methacrylate, most preferably of a mixture of 15-25 wt.-% of methacrylic acid, 8-12 wt.-% of ethyl acrylate and 65-75 wt.-% of n-butyl methacrylate, such as even more in particular of 18-23 wt.-% of methacrylic acid, 9-11 wt.-% of ethyl acrylate and 67-72 wt.-% of n-butyl methacrylate.

In all embodiments of the present invention, the total amount of UV-filter substances in the topical sunscreen compositions according to the present invention is preferably selected in the range of 1 to 40 wt.-%, such as more preferably in the range of 5 to 35 wt.-%, such as most preferably in the range of 10 to 30 wt.-%, based on the total weight of the topical sunscreen composition.

Suitable UV-filter substances according to the present invention are UVA, UVB and/ or broadspectrum UV-filter substances which are or can be used as cosmetically acceptable UVA, UVB or broadspectrum UV-filter substances. Such UV-filter substances are e.g. listed in the CTFA Cosmetic ingredient Handbook or "The Encyclopedia of Ultraviolet Filters" (ISBN: 978-1-932633-25-2) by Nadim A. Shaath.

The UV-filter substances may be organic or inorganic compounds. Exemplary UVA, UVB and/ or broadspectrum UV-filter substances encompass dibenzoylmethane derivatives such as e.g. butyl methoxydibenzoylmethane (PARSOL^{®} 1789); acrylates such as e.g. octocrylene (PARSOL^{®} 340); camphor derivatives such as e.g. 4-methyl benzylidene camphor (PARSOL^{®} 5000) or terephthalylidene dicamphor sulfonic acid (Mexoryl^{®} SX); cinnamate derivatives such as e.g. ethylhexyl methoxycinnamate (PARSOL^{®} MCX) or isoamyl methoxycinnamate; p-aminobenzoic acid derivatives such as e.g. p-aminobenzoic acid or 2-ethylhexyl p-dimethylaminobenzoate; benzophenones such as e.g. benzophenone-3, benzophenone-4, 2,2',4,4'-tetrahydroxy-benzophenone or 2,2'-dihydroxy-4,4'-dimethoxybenzophenone; esters of benzalmalonic acid such as e.g. d i-(2-ethylhexyl) 4-methoxybenzalmalonate; organosiloxane compounds carrying chromophore groups such as e.g. polysilicone-15 (PARSOL^{®} SLX) or drometrizole trisiloxane (Mexoryl^{®} XL); imidazole derivatives such as e.g. 2-phenyl benzimidazole sulfonic acid and salts thereof such as e.g. its sodium- or potassium salts (PARSOL^{®} HS); salicylate derivatives such as e.g. ethylhexyl salicylate (PARSOL^{®} EHS, Neo Heliopan^{®} OS), isooctyl salicylate or homosalate (PARSOL^{®} HMS, Neo Heliopan^{®} HMS); triazine derivatives such as e.g. ethylhexyl triazone (Uvinul^{®} T-150), diethylhexyl butamido triazone (Uvasorb^{®} HEB), bis-ethylhexyloxyphenol methoxyphenyl triazine (Tinosorb^{®} S) or 2,4,6-tris[1,1'-biphenyl]-4-yl-1,3,5-triazine [CAS No. 31274-51-8]; Benzotriazole derivatives such as e.g. methylene bis-benzotriazolyl tetramethylbutylphenol (Tinosorb^{®} M); encapsulated UV-filters such as e.g. encapsulated ethylhexyl methoxycinnamate (Eusolex^{®} UV-pearls); amino substituted hydroxybenzophenones such as e.g. diethylamino hydroxybenzoyl hexyl benzoate (Aminobenzophenon, Uvinul^{®} A Plus); benzoxazol-derivatives such as e.g. 2,4-bis-[5-1(dimethylpropyl)benzoxazol-2-yl-(4-phenyl)-imino]-6-(2-ethylhexyl)-imino-1,3,5-triazin (Uvasorb^{®} K2A); phenylene-1,4-bis-benzimidazolsulfonic acids or salts thereof such as e.g. disodium phenyl dibenzimidazole tetrasulfonate (2,2-(1,4-phenylene)bis-(1H-benzimidazol-4,6-disulfonic acid, Neoheliopan^{®} AP); 1,1'-(1,4-piperazinediyl)bis[1-[4-(diethylamino)-2-hydroxybenzoyl]phenyl]-methanone (CAS No. 919803-06-6); as well as Bis(butylbenzoate) diaminotriazine aminopropyltrisiloxane (CAS No. 207562-42-3).

Inorganic U V-filter substances encompass pigments such as e.g. microparticulated zinc oxide or titanium dioxide (e.g. commercially available as PARSOL^{®}TX) The term "microparticulated" refers to a particle size from about 5 nm to about 200 nm, particularly from about 15 nm to about 100 nm. The particles may also be coated by other metal oxides such as e.g. aluminum or zirconium oxides or by organic coatings such as e.g. polyols, methicone, aluminum stearate, alkyl silane. Such coatings are well known in the art.

Preferred UVB-filter substances to be incorporated into the topical sunscreen compositions according to the present invention encompass polysilicone-15, phenylbenzimidazol sulfonic acid, octocrylene, ethylhexyl methoxycinnamate, ethyl hexylsalicylate and/ or homosalate.

Preferred broadband UV-filter substances to be incorporated into the topical sunscreen compositions according to the present invention encompass unsymmetrical s-triazine derivatives such as in particular bis-ethylhexyloxyphenol methoxyphenyl triazine; certain benzophenones such as e.g. 2-hydroxy-4-methoxy-benzophenon; methylene bis-benzotriazolyl tetramethylbutylphenol, and/ or titanium dioxide.

Preferred UVA-filter substances to be incorporated into the topical sunscreen compositions according to the present invention encompass butyl methoxydibenzoylmethane; diethylamino hydroxybenzoyl hexyl benzoate; 2,4-bis-[5-1(dimethylpropyl)benzoxazol-2-yl-(4-phenyl)-imino]-6-(2-ethylhexyl)-imino-1,3,5-triazine and/ or disodium phenyl dibenzimidazole tetrasulfonate; in particular butyl methoxydibenzoylmethane and/ or diethylamino hydroxybenzoyl hexyl benzoate.

If the topical sunscreen compositions comprise butyl methoxydibenzoylmethane, then they advantageously contain in addition at least one suitable photostabilizer for butyl methoxydibenzoylmethane. Besides specific UV-filters listed above which are known to a person skilled in the art to be able to photostabilize butyl methoxydibenzoylmethane, further exemplary photostabilizers encompass Polyester 8 (Polycrylene^{®}); Methoxycrylene (Solastay); diethylhexyl syringylidene malonate (Oxynex ST liquid); diethylhexyl naphthalate (Corapan TQ) as well as Benzotriazolyl Dodecyl p-Cresol (Tinogard^{®} TL) without being limited thereto. An overview on such photostabilizers is e.g. given in 'SPF Boosters & Photostability of Ultraviolet Filters', HAPPI, October 2007, p. 77-83 which is included herein by reference. These photostabilizers are generally used in an amount of 0.05 to 10 wt.-% with respect to the total weigh of the topical sunscreen composition.

In a particular preferred embodiment the topical sunscreen compositions according to the present invention comprise at least 2, more preferably at least 3, most preferably at least 4 different UV-filter substances. Advantageously, the topical sunscreen compositions according to the present invention, in addition, comprise at least one UV-B filter substance and at least one UVA-filter substance.

In a particular advantageous embodiment, the at least one UV-filter substances present in the topical sunscreen compositions according to the present invention are selected from the group consisting of polysilicone-15, phenylbenzimidazol sulfonic acid, octocrylene, ethylhexyl methoxycinnamate, ethyl hexylsalicylate, homosalate, bis-ethylhexyloxyphenol methoxyphenyl triazine, methylene bis-benzotriazolyl tetramethylbutylphenol, titanium dioxide, butyl methoxydibenzoylmethane, diethylamino hydroxybenzoyl hexyl benzoate and/ or disodium phenyl dibenzimidazole tetrasulfonate as well as mixtures thereof, preferably the at least one UV-filter substances is selected from the group consisting of butyl methoxydibenzoylmethane, phenyl benzimidazol sulfonic acid, polysilicone-15, octocrylene, homosalate, and ethylhexyl salicylate as well as mixtures thereof. In a particular embodiment, the topical sunscreen compositions according to the present invention comprise a mixture of butyl methoxydibenzoylmethane, phenylbenzimidazol sulfonic acid, octocrylene, homosalate, and ethylhexyl salicylate or butyl methoxydibenzoylmethane, phenylbenzimidazol sulfonic acid, polysilicone-15, octocrylene, and ethylhexyl salicylate as sole UV-filter substance.

The UV-filter substances are incorporated either in the aqueous or in the lipophilic part of the topical sunscreen compositions, depending if they are water or oil (fat) soluble/ miscible UV filter substances or may even be added to the final emulsion by standard methods known to a person skilled in the art.

The acrylic emulsion polymers according to the invention are prepared by emulsion polymerization methods according to known methods as e.g. described in WO 2012072774.

The acrylic emulsion polymers according to the present invention preferably have a molecular weight between 30-500 kDalton, more preferably 50 - 250 kDalton and most preferred between 75 and 200 kDalton such as in the range of 100 to 150 kDalton, and a glass transition temperature (Tg) between 40 and 140°C, more preferably between 55 and 130°C and most preferred between 70-120 °C such as e.g. between 70 and 100 °C. Advantageously the emulsion polymers according to the invention have a molecular weight between 75 -200 kDalton and a Tg in the range of 70-120 °C such as in particular a molecular weight between 100-150 kDalton and a Tg in the range of 70-100 °C.

Preferably, the acrylic emulsion polymer is used in the form of an aqueous dispersion, wherein the dispersion has a polymer solid content in the range of 30 to 60 wt.-%, such as in particular in the range of 35 to 45 wt.-%. In a preferred embodiment this dispersion further contains methylisothiazolinone, in particular in an amount of about 30 to 50 ppm. In a very particular embodiment the aqueous dispersion of the acrylic emulsion polymer according to the invention has a polymer solid content in the range of 35 to 45 wt.-% such as 39-40 wt.-% and a methylisothiazolinone content of about 50 ppm.

A particular suitable acrylic emulsion polymer according to the present invention is available at DSM Nutritional Products Ltd. under the Tradename TILAMAR^{®} Fix A140 (INCI: acrylates copolymer, Chemical Name: polymer with 2-methyl-2-propenoic acid, butyl 2-methyl-2-propenoate, and ethyl 2-propenoate, CAS Number: 26715-43-5).

The term "topical" as used herein is understood to mean external application to keratinous substances, such as in particular the skin around the eyes.

As the topical sunscreen compositions according to the invention are intended for topical application, they comprise a cosmetically acceptable carrier i.e. a physiologically acceptable medium which is compatible with keratinous substances, such as in particular the skin. Thus, the term cosmetically acceptable carrier refers to all cosmetic carriers and/or excipients and/or diluents conventionally used in topical sunscreen compositions.

The topical sunscreen compositions of the invention can also contain further conventional cosmetic adjuvants and additives, such as preservatives/antioxidants, fatty substances/oils, water, organic solvents, silicones, thickeners, softeners, emulsifiers, antifoaming agents, aesthetic components such as fragrances, surfactants, fillers, sequestering agents, anionic, cationic, nonionic or amphoteric polymers or mixtures thereof, propellants, acidifying or basifying agents, dyes, colorings/colorants, abrasives, absorbents, chelating agents and/ or sequestering agents, essential oils, skin sensates, astringents, pigments or any other ingredients usually formulated into such compositions.

In accordance with the present invention, the topical sunscreen compositions according to the invention may comprise further cosmetically active ingredients conventionally used in topical sunscreen compositions. Exemplary active ingredients encompass skin lightening agents; agents for the treatment of hyperpigmentation; agents for the prevention or reduction of inflammation; firming, moisturizing, soothing, and/ or energizing agents as well as agents to improve elasticity and skin barrier.

Examples of cosmetic carriers, excipients, ingredients, adjuvants, diluents and additives commonly used in the skin care industry, which are suitable for use in the compositions of the present invention, are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The necessary amounts of the active ingredients as well as the cosmetic adjuvants, diluents and additives can, based on the desired product form and application, easily be determined by the skilled person. The additional ingredients can either be added to the oily phase, the aqueous phase or separately as deemed appropriate.

The cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

The topical sunscreen compositions according to the present invention may be in the form of a suspension or dispersion in solvents or fatty substances, or alternatively in the form of an emulsion or micro emulsion (in particular of oil-in-water (O/W) or water-in-oil (W/O) type, silicone-in-water (Si/W) or water-in-silicone (W/Si) type, PIT-emulsion, multiple emulsion (e.g. oil-in-water-in oil (O/W/O) or water-in-oil-in-water (W/O/W) type), pickering emulsion, hydrogel, alcoholic gel, lipogel, one- or multiphase solution or vesicular dispersion or other usual forms, which can also be applied by pens, as masks or as sprays.

If the topical sunscreen composition is an emulsion such as in particular an O/W, W/O, Si/W, W/Si O/W/O, W/O/W multiple or a pickering emulsion, then the amount of the oily phase present in such topical sunscreen emulsions is preferably at least 10 wt.-%, such as in the range of 10 to 60 wt.-%, preferably in the range of 20 to 50 wt.-%, most preferably in the range of 25 to 40 wt.-%, based on the total weight of the topical sunscreen composition.

The topical sunscreen compositions according to the invention can be provided, for example, in the form of a serum, a milk, a lotion, a hydrodispersions, a foundation, a cream, a creamgel, or a gel, which are prepared according to the usual methods.

In one embodiment, the topical compositions according to the present invention are advantageously in the form of an oil-in-water (O/W) emulsion comprising an oily phase dispersed in an aqueous phase in the presence of an O/W emulsifier. The preparation of such O/W emulsions is well known to a person skilled in the art.

If the topical composition according to the invention is an O/W emulsion, then it contains advantageously at least one O/W- or Si/W-emulsifier selected from the list of, Glyceryl Stearate Citrate, Glyceryl Stearate SE (self-emulsifying), stearic acid, salts of stearic acid, polyglyceryl-3-methylglycosedistearate. Further suitable emulsifiers are phosphate esters and the salts thereof such as cetyl phosphate (e.g. as Amphisol^{®} A from DSM Nutritional Products Ltd.), diethanolamine cetyl phosphate (e.g. as Amphisol^{®} DEA from DSM Nutritional Products Ltd.), potassium cetyl phosphate (e.g. as Amphisol K from DSM Nutritional Products Ltd.), sodiumcetearylsulfat, sodium glyceryl oleate phosphate, hydrogenated vegetable glycerides phosphate and mixtures thereof. Further suitable emulsifiers are sorbitan oleate, sorbitan sesquioleate, sorbitan isostearate, sorbitan trioleate, Cetearyl Glucoside, Lauryl Glucoside, Decyl Glucoside, Sodium Stearoyl Glutamate, Sucrose Polystearate and Hydrated Polyisobuten. Furthermore, one or more synthetic polymers may be used as an emulsifier. For example, PVP eicosene copolymer, acrylates/C10-30 alkyl acrylate crosspolymer, and mixtures thereof.

The at least one O/W, respectively Si/W emulsifier is preferably used in an amount of 0.5 to 10 wt. %, in particular in the range of 0.5 to 6 wt.-% such as more in particular in the range of 0.5 to 5 wt.-% such as most in particular in the range of 1 to 4 wt.-%, based on the total weight of the topical sunscreen composition.

Particular suitable O/W emulsifiers to be used in the topical sunscreen compositions according to the invention encompass phosphate esters emulsifier such as advantageously 8-10 Alkyl Ethyl Phosphate, C9-15 Alkyl Phosphate, Ceteareth-2 Phosphate, Ceteareth-5 Phosphate, Ceteth-8 Phosphate, Ceteth-10 Phosphate, Cetyl Phosphate, C6-10 Pareth-4 Phosphate, C12-15 Pareth-2 Phosphate, C12-15 Pareth-3 Phosphate, DEA-Ceteareth-2 Phosphate, DEA-Cetyl Phosphate, DEA-Oleth-3 Phosphate, Potassium cetyl phosphate, Deceth-4 Phosphate, Deceth-6 Phosphate and Trilaureth-4 Phosphate.

A particular suitable O/W emulsifier to be used in the topical sunscreen compositions according to the invention is potassium cetyl phosphate e.g. commercially available as Amphisol^{®} K at DSM Nutritional Products Ltd Kaiseraugst.

Another particular suitable class of O/W emulsifiers are non-ionic self-emulsifying system derived from olive oil e.g. known as (INCI Name) cetearyl olivate and sorbitan olivate (Chemical Composition: sorbitan ester and cetearyl ester of olive oil fatty acids) sold under the tradename OLIVEM 1000.

In particular embodiment, the invention relates to topical sunscreen compositions with all the definitions and preferences given herein in the form of O/W emulsions comprising an oily phase dispersed in an aqueous phase in the presence of an O/W emulsifier wherein the O/W emulsifier is potassium cetyl phosphate. The amount of oily phase in such O/W emulsions is preferably at least 10 wt.-%, more preferably in the range of 10 to 60 wt.-%, most preferably in the range of 20 to 50 wt.-%, such as in the range of 25 to 40 wt.-%.

The topical sunscreen compositions according to the invention in general have a pH in the range of 3 to 10, preferably a pH in the range of 4 to 8 and most preferably a pH in the range of 4 to 7.5. The pH can easily be adjusted as desired with suitable acids such as e.g. citric acid or bases such as sodium hydroxide (e.g. as aqueous solution), Triethanolamine (TEA Care), Tromethamine (Trizma Base) and Aminomethyl Propanol (AMP-Ultra PC 2000) according to standard methods in the art.

The amount of the topical sunscreen composition to be applied to the skin is not critical and can easily be adjusted by a person skilled in the art. Preferably the amount is selected in the range of 0.1 to 3 mg/ cm2 skin, such as preferably in the range of 0.1 to 2 mg/ cm2 skin and most preferably in the range of 0.5 to 2 mg / cm2 skin.

The invention is further illustrated with reference to the following, non-limiting examples, in which all percentages are by weight based on total weight unless otherwise specified.

### Example 1: Water resistance

0.75 mg/cm² of the respective compositions as outlined in table1 were applied to PMMA plates and the plates were dried at RT for 30 min. Afterwards the initial in vitro SPF (Sun Protection Factor) were determined with a "m.u.t HELIAS fiber optical integrator". Then the plates were immersed into a flask filled with 2.5 l water (bi-distilled) for 20 min while the water was stirred with a magnetic stirrer at 450 1/min at RT (The plates were attached at the edge of the flask with a clothespin, such that the side covered with the composition was directed into the flask). Afterwards the plates were dried at 40 °C for 30 min. The immersion/ drying procedure was repeated once. After final drying the in-vitro SPF was measured again and the water resistance was calculated as % WR = [(SPF after immersion - 1/ (SPF initial - 1)]*100 and the Δ water resistance as (% WR of sample with TILAMAR FIX A140) - (% WR of Sample without TILAMAR FIX A140) Each composition was tested separately on 4 plates.

The water resistance for each composition was determined as mean value from the 4 plates.
R-1 and R-2 = Respective reference example without TILAMAR FIX A140
I-1 and I-2 = Examples according to the invention
R-3 and R-4 = References with other acrylates co-polymers

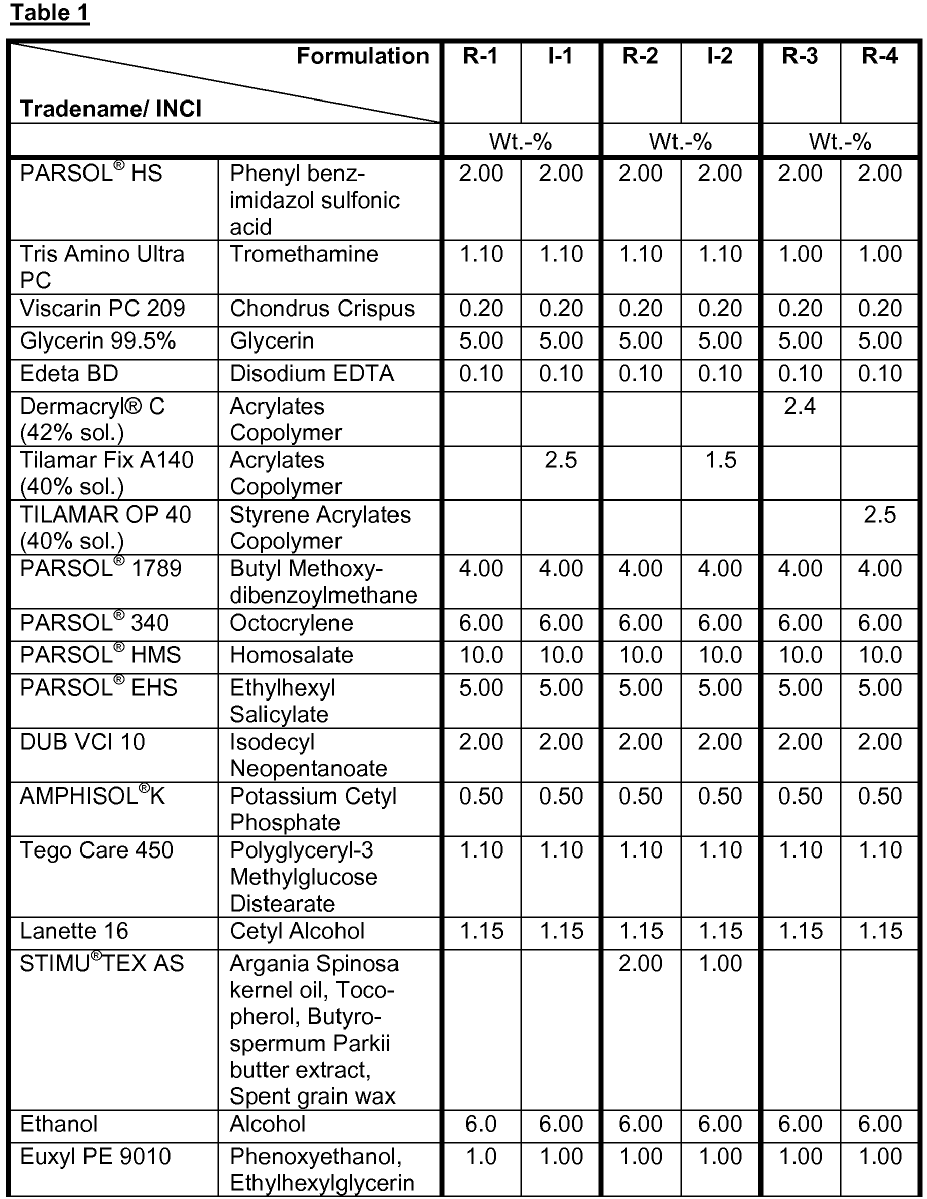

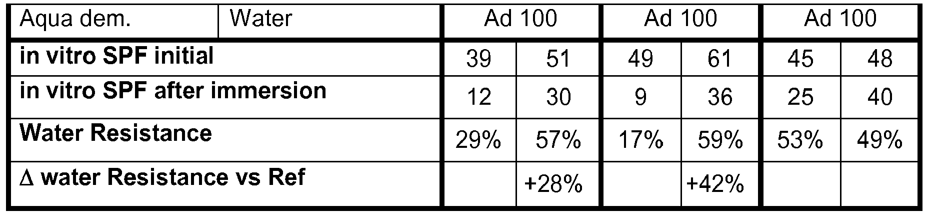

As can be retrieved from table 1, the water resistance is highly improved by the addition of TILAMAR FIX A140.

### Example 2: in vivo boosting

The in-vivo SPF of the formulations as outlined in table 2 was determined according to the COLIPA protocol (EU Commission Recommendation of 22 September 2006 on the efficacy of sunscreen products and the claims relating thereto (2005/647/EC)) and ISO 24444:2010: In vivo determination of the sun protection factor (SPF) on 10 volunteers (24-51 years old, Phototype I, II or III).

**Table 2**

| **Ingredients** | **INCI Name** | **Wt.-%** | |
|---|---|---|---|
| Lanette 18 | STEARYL ALCOHOL | 2.50 | 2.50 |
| PARSOL^{®} 340 | OCTOCRYLENE | 8.00 | 8.00 |
| PARSOL^{®} 1789 | BUTYL METHOXYDIBENZOYLMETHANE | 4.00 | 4.00 |
| PARSOL^{®} SLX | POLYSILICONE-15 | 2.00 | 2.00 |
| PARSOL^{®} EHS | ETHYLHEXYL SALICYLATE | 4.00 | 4.00 |
| Finsolv TN | C12-15 ALKYL BENZOATE | 3.00 | 3.00 |
| Cetiol C5 | COCO-CAPRYLATE | 3.00 | 3.00 |
| Dow Corning 200 Fluid 100 CST | DIMETHICONE | 2.00 | 2.00 |
| Euxyl PE 9010 | PHENOXYETHANOL, ETHYLHEXYLGLYCERIN | 1.00 | 1.00 |
| VALVANCE Touch 150 | METHYL METHACRYLATE CROSSPOLYMER | 2.00 | 2.00 |
| Sepinov EMT 10 | HYDROXYETHYL ACRYLATE/SODIUM ACRYLOYLDIMETHYL TAURATE COPOLYMER | 1.30 | 1.30 |
| WATER DEM. | AQUA | 48.34 | 48.34 |
| Edeta BD | DISODIUM EDTA | 0.10 | 0.10 |
| TILAMAR^{®} FIX A140 40% emulsion | ACRYLATES COPOLYMER, AQUA | 0.60 | - |
| Sodium Hydroxide | SODIUM HYDROXIDE | 0.03 | 0.03 |
| 1,3-Butylenglycol | BUTYLENE GLYCOL | 3.00 | 3.00 |
| Keltrol CG-T | XANTHAN GUM | 0.20 | 0.20 |
| AMPHISOL^{®} K | POTASSIUM CETYL PHOSPHATE | 2.00 | 2.00 |
| WATER DEM. | AQUA | Ad 100 | Ad 100 |
| Sodium Hydroxide | SODIUM HYDROXIDE | 0.30 | 0.30 |
| PARSOL^{®} HS | PHENYLBENZIMIDAZOLE SULFONIC ACID | 2.00 | 2.00 |
| Citric Acid 25% solution | CITRIC ACID, AQUA | 0.23 | 0.23 |
| D-Panthenol 75 L | PANTHENOL, AQUA | 0.10 | 0.10 |
| PENTAVITIN^{®} | SACCHARIDE ISOMERATE, AQUA, CITRIC ACID, SODIUM CITRATE | 0.30 | 0.30 |
| In vivo SPF | | 40 | 34 |

As can be retrieved from table 2, the in vivo SPF is significantly enhanced by the addition of TILAMAR FIX A 140.

### Example 3: Sensory test

The formulations I-1 and R-4 as outlined in Table 1 above were tested in a blind study with a trained sensorial panel consisting of 8 persons under the following conditions:
The evaluation takes part on the inner forearm; the panel leader applies 50 µL of the respective sample.
Evaluator spreads the product within a defined circle of 5 cm diameter using index or middle finger, circular motion, rate of 2 rotations/second. This is the so called rub-out phase. After the rub-out phase the stickiness is assessed relative to a set of standards. The stickiness is scaled on a scale from 0 to 100 in comparison to trained standards. Data are median values. The stickiness values are assessed immediately after application as well as after 20 min. As can be retrieved from table 4, the composition according to the present invention showed a significantly reduced stickiness directly after application as well as after 20 min compared to R-4 comprising a styrene acrylates copolymer.

**Table 4**

| **Sample** | **Stickiness** | |
|---|---|---|
| | Immediate | After 20 min |
| **I-1** | 11 | 5 |
| **R-4** | 17 | 8 |

## Claims

1. A topical sunscreen composition comprising at least one UV-filter substance in an amount of at least 1 wt.-%, wherein the topical sunscreen composition further comprises an effective amount of an acrylic emulsion polymer obtained by emulsion polymerization of a monomer composition consisting of a mixture of methacrylic acid, ethyl acrylate and n-butyl methacrylate.

2. The topical sunscreen composition according to claim 1, wherein the effective amount of the acrylic emulsion polymer is selected in the range of 0.1-3 wt.-%, based on the total weight of the topical sunscreen composition.

3. The topical sunscreen composition according to claim 1 or 2, wherein the monomer composition consists of a mixture of 10-30 wt.-% of methacrylic acid, 5-15 wt.-% of ethyl acrylate and 60-80 wt.-% of n-butyl methacrylate.

4. The topical sunscreen composition according to any one of claim 1 to 3, wherein the amount of the at least one UV-filter substance is selected in the range of 5 to 35 wt.-%.

5. The topical sunscreen composition according to any one of claim 1 to 4, wherein at least one UVB- and at least one UVA-filter substance is present in the topical sunscreen composition.

6. The topical sunscreen composition according to any one of claim 1 to 5, wherein the at least one UV-filter substance is selected from the group consisting of polysilicone-15, phenylbenzimidazol sulfonic acid, octocrylene, ethylhexyl methoxycinnamate, ethyl hexylsalicylate, homosalate, bis-ethylhexyloxyphenol methoxyphenyl triazine, methylene bis-benzotriazolyl tetramethylbutylphenol, titanium dioxide, butyl methoxydibenzoylmethane; diethylamino hydroxybenzoyl hexyl benzoate and/ or disodium phenyl dibenzimidazole tetrasulfonate as well as mixtures thereof.

7. The topical sunscreen composition according to claim 6, wherein the at least one UV-filter substance is selected from the group consisting of butyl methoxydibenzoylmethane, phenylbenzimidazol sulfonic acid, polysilicone-15, octocrylene, homosalate, and ethylhexyl salicylate as well as mixtures thereof.

8. The topical sunscreen composition according to any one of claims 1 to 7, wherein the acrylic emulsion polymer is used in the form of an aqueous dispersion having a polymer solid content in the range of 30 to 60 wt.-%.

9. The topical sunscreen composition according to any one of claims 1 to 8, wherein topical sunscreen composition is in the form of an oil-in-water (O/W) emulsion comprising an oily phase dispersed in an aqueous phase in the presence of an O/W emulsifier.

10. The topical sunscreen composition according to claim 9, wherein the O/W emulsifier is potassium cetyl phosphate.

11. The topical sunscreen composition according to claim 9 or 10, wherein the oily phase constitutes from 10 to 60 wt.-%, based on the total weight of the topical sunscreen composition.

12. Use of an acrylic emulsion polymer obtained by emulsion polymerization of a monomer composition consisting of a mixture of methacrylic acid, ethyl acrylate and n-butyl methacrylate for increasing the water resistance and or SPF of a topical sunscreen composition comprising at least one UV-filter substance.

13. A method for increasing the water resistance and/ or the SPF of at least one UV-filter substance in a topical sunscreen composition, said method comprising the addition of an acrylic emulsion polymer obtained by emulsion polymerization of a monomer composition consisting of a mixture of methacrylic acid, ethyl acrylate and n-butyl methacrylate into said topical sunscreen composition and, preferably, observing or appreciating the result.
